# EUROPEAN PATENT SPECIFICATION

(11) **EP 4 090 314 B1**
(45) Date of publication and mention of the grant of the patent: **31.12.2025**
(21) Application number: 21701923.1
(22) Date of filing: 14.01.2021
(51) Int. Cl.: A61K 9/00, A61K 31/155, A61K 9/24, A61P 25/34

(54) **LOZENGE**
LUTSCHTABLETTE
PASTILLE

(30) Priority: 15.01.2020 SE 2050030
(43) Date of publication of application: 23.11.2022
(73) Proprietor: McNeil AB, 25109 Helsingborg (SE)
(72) Inventor: THYRESSON, Kristina, 22657 Lund (SE); LINDELL, Katarina, 25109 Helsingborg (SE)
(74) Representative: Carpmaels & Ransford LLP
(86) International application number: PCT/EP2021/050705
(87) International publication number: WO 2021/144367

(56) References cited:
- WO-A1-02/076211
- WO-A1-03/039518
- WO-A1-2019/244176
- US-A1- 2010 247 586

## Description

### FIELD OF THE INVENTION

This invention relates to a nicotine lozenge, as defined in the appended claims, giving an immediate release and uptake of nicotine and an extended release and uptake of nicotine as well as describing suitable manufacturing processes for such lozenge formulations. The invention also relates to the lozenge for use in the treatment of a human being suffering from cravings from tobacco and/or e-cigarette dependency.

### BACKGROUND OF INVENTION

According to WHO about six million people die from smoking related diseases each year, even though there are products (medicines) on the market to help a smoker to quit smoking; products such as e.g. nicotine comprising chewing gums, lozenges, sprays and transdermal patches.

One traditional way to produce a lozenge is to create a complex of nicotine with a cation exchange resin and to add this complex to a lozenge formulation.

Such lozenges are available on the market since many years, sold under, for example the trade mark Nicorette^{®}. However, there are consumers that are looking for nicotine products (medicines) that could provide faster craving relief, closer to the craving relief of a cigarette and thus there is still an opportunity to develop new nicotine lozenges that could satisfy this population of consumers using tobacco and/or e-cigarettes.

One product on the market giving rise to a faster craving relief is the Nicorette^{™} QuickMist^{™}, a mouth spray to be applied to the oral mucosa, from which the nicotine compound is readily absorbed into the blood stream to give a fast craving relief.

WO2008140372 discloses a lozenge having nicotine in the core and with a coating wherein there is one or more amino acids in the coatings that acts as a buffer. The application is silent about nicotine in another portion/layer.

WO2007133141 discloses different formats wherein the examples disclose chewing gum and tablet. None of the examples discloses that nicotine should be present in two parts of the format.
WO 03/039518 relates to *"new oral dosage formulations comprising a nicotine active, optionally combined with an antidepressant, which through the controlled release of the active ingredient(s) alleviate some of the nicotine withdrawal symptoms a person may experience during attempts to quit smoking".*
WO 02/076211 relates to *"glassy matrix solid oral dosage forms useful for transmucosal oral administration of a nicotine active".*
WO 2019/244176 relates to *"stable oral lozenges containing nicotine active which is bioequivalent to approved marketed lozenges as nicotine replacement therapy for reducing or stopping tobacco usage".*
US 2010/247586 relates to a *"multi portion intra-oral dosage form"* having *"at least one pharmaceutically active or health promoting agent with at least one portion"* comprising *"a component for creating a noticeable organoleptic sensation".*

However, there are consumers who could benefit from products that could provide both a fast and more prolonged craving relief from one and the same product.

### SUMMARY OF THE INVENTION

The invention is defined in the appended claims.

The inventors have been exploring the possibility to create a lozenge giving rise to an immediate as well as an extended release and uptake of nicotine, while the lozenge still has good taste and palatability. To provide an immediate release and uptake from a nicotine lozenge product, the inventors approach has been to apply nicotine in a more readily available nicotine salt form, such as nicotine bitartrate or nicotine ditartrate dihydrate comprised in at least one outer layer/portion on the surface of a nicotine lozenge.

The pKa for nicotine is approximately 7.8. It is well known that the uncharged (free base form) could more easily and faster enter biological membranes, such as the oral mucosa, to obtain a fast systemic uptake, compared to the nicotine in its positively charged acid form.

Thus, if an acid drug salt like nicotine bitartrate is co-formulated in a product in such a way that the product also contains some readily available and fast released buffer(s), that are present in at least one film coating or at least one portion/layer on a lozenge, the buffer(s) can rapidly and transiently increase the pH of the solvent (in this case saliva) in order to convert nicotine into its' free base form resulting in a faster absorption of the nicotine released from at least one coating or portion/layer on the surface of a nicotine lozenge.

The average pH of the human saliva is normally just about 6-7.5.

By selecting and adding some readily and fast released buffer(s) to the at least one coating, or at least one portion/layer, a fast pH increase of the saliva of approximately one pH unit above the pKa of nicotine (thus in the range of at or above pH 9) could be achieved. This would result in that approximately about 90 % of the nicotine disintegrated and dissolved from at least one portion/layer will be in its' free base form and immediately be absorbed into the systemic circulation to provide for a faster craving relief.

To be able to provide a transient and suitable pH increase there is a benefit of using a buffering system, such as one or more different buffer(s) (buffer species). Normally, buffering systems do not taste well and thus they need to be selected carefully and taste masked by e.g. sweeteners and flavors. In addition, the buffering capacity of one buffer (buffer species) might not be sufficient.

Nicotine free base form is not suitable to directly be formulated into semisolid or solid dosage forms since the nicotine base form is a highly volatile liquid at normal conditions.

To provide an extended release of nicotine from a nicotine lozenge, nicotine bound to a resin such as nicotine polacrilex is used. Nicotine polacrilex is normally used in the medicated lozenges available on the market and it provides an extended release over a time up to about 10 to 20 minutes, depending on the usage.

The invented nicotine lozenge provides new features in one and the same product, compared to currently available commercialized medicinal nicotine products.

The nicotine lozenge could also become an attractive product for consumers or patients that prefer nicotine lozenges as a format.

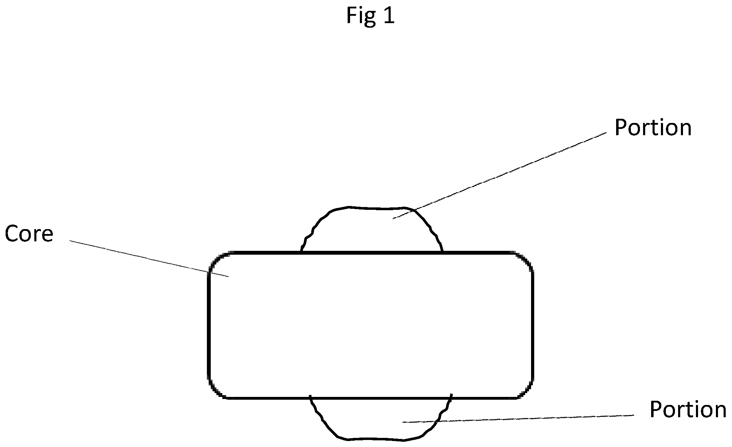

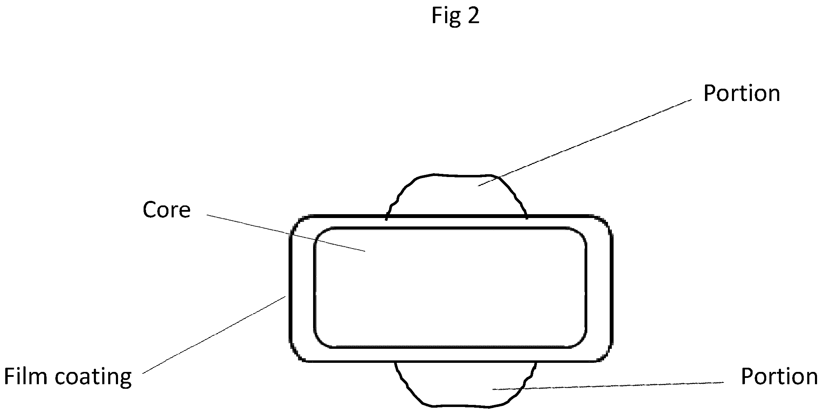

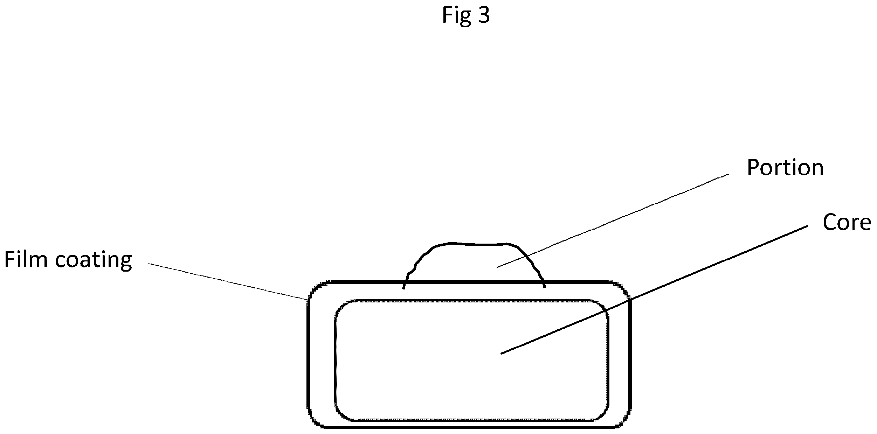

In a first aspect the invention relates to a lozenge comprising nicotine in the core and
a) at least a first portion/layer comprising at least one buffer and at least one sugar alcohol or a mixture of sugar alcohols fused onto the outside of the lozenge, and
b) at least a second portion/layer comprising nicotine salt and at least one sugar alcohol or a mixture of sugar alcohols fused onto the lozenge, (see fig 1)
   or
c) at least one polymer-based film coating covering the lozenge comprising at least one buffer and at least one flavor and at least one sweetener, and
d) at least one portion/layer comprising nicotine salt and at least one sugar alcohol or a mixture of sugar alcohols fused onto the lozenge, (see Fig3)
   or
e) at least one polymer-based film coating covering the lozenge comprising at least one flavor and at least one sweetener, and
f) at least a first portion/layer comprising at least one buffer and at least one sugar alcohol or a mixture of sugar alcohols fused onto the outside of the lozenge, and
g) at least a second portion/layer comprising nicotine salt and at least one sugar alcohol or a mixture of sugar alcohols fused onto the lozenge, (see Fig 2)
   and wherein the at least one sugar alcohol or mixture of sugar alcohols comprised in the portion(s)/layer(s) comprises at least erythritol,
   and wherein the nicotine release from the at least one portion/layer is immediate and the nicotine release from the lozenge is extended
      and
   wherein the at least one buffer promotes a rapid uptake of nicotine through the oral mucosa.

Also described is a manufacturing process for the production of such a lozenge.

In a final aspect the invention relates to the lozenge for use in the treatment of a human being suffering from cravings from tobacco dependency and/or e-cigarette dependency, i.e., to help a human quitting smoking.

### BRIEF DESCRIPTION OF THE DRAWINGS

There is no scaling in the drawings, and they are there to illustrate the invention.
Fig 1 shows one embodiment of a nicotine lozenge according to the invention having a core and two portions.
Fig 2 shows one embodiment of a nicotine lozenge according to the invention having a core, a film coating and two portions.
Fig 3 shows one embodiment of a nicotine lozenge according to the invention having a core, a film coating and one portion.
Fig 4 shows one embodiment of a nicotine lozenge according to the invention having a core, a film coating and two portions.

### DETAILED DESCRIPTION OF EMBODIMENTS OF THE INVENTION

### Definitions

In the context of the present application and invention the following definitions apply:

The term "nicotine" refers to the amount (mg) of nicotine in any salt form or bound to any carrier calculated as the amount of corresponding free base per piece of lozenge.

The term "portion/layer" is intended to mean a part that is fused or attached onto the lozenge at any place. It might be in any kind of form including a round portion (dot), square portion, conic portion, triangle etc. The portion may be in the form of a trade mark as well as having a color. The portion may also be a layer present on the surface of the lozenge, such as 40 % of the lozenge surface for one layer, such as one side, or both sides of the lozenge. The et least one portion/layer may be deposited in a debossed smooth cavity(cavities) on the lozenge.

The term "buffer(s)" refers to two different kinds of buffer species also differentiating the corresponding acid-base pair of a buffer system.

As used herein, the term "extended release" ("ER") refers to formulations which are characterized by that the nicotine present in the lozenge will be released over an extended period of sucking, normally for 10-20 minutes, the time a consumer or patient is sucking on (using) the lozenge. The release profile may be assessed via *in vitro* dissolution using techniques well known for a person skilled in the art.

The term "immediate release" ("IR") as used herein is intended to mean the release of the nicotine comprised in the portion(s)/layer(s) on the surface of the lozenge. The released nicotine is aimed to be available for fast oromucosal absorption. The rate of release of nicotine is not prolonged by means of a controlled release matrix or other such means but it is dependent of the disintegration and dissolution of the portion(s)/layer(s) and water solubility of the polyol, polymer, nicotine salt and buffer(s). As described herein, an "immediate release" component is released shortly after the sugar alcohol and nicotine mixture is disintegrated and dissolved (in the saliva) which occurs shortly after the sucking on the lozenge begins.

The calculation of the amount of nicotine present in the lozenge is calculated and most often expressed as the amount of the corresponding free base form of nicotine.

The term "fused onto" used throughout the application is intended to be interchangeable with "attached to", "fused to", "sticked to", "deposited onto", "applied to", "adhered to", or "melted onto" or "printed on".

### LOZENGE

In one embodiment the invention relates to a lozenge comprising nicotine in the core and
a) at least a first portion/layer comprising at least one buffer and at least one sugar alcohol or a mixture of sugar alcohols fused onto the outside of the lozenge, and
b) at least a second portion/layer comprising nicotine salt and at least one sugar alcohol or a mixture of sugar alcohols fused onto the lozenge, (see fig 1)
   or
c) at least one polymer-based film coating covering the lozenge comprising at least one buffer and at least one flavor and at least one sweetener, and
d) at least one portion/layer comprising nicotine salt and at least one sugar alcohol or a mixture of sugar alcohols fused onto the lozenge, (see Fig3)
   or
e) at least one polymer-based film coating covering the lozenge comprising at least one flavor and at least one sweetener, and
f) at least a first portion/layer comprising at least one buffer and at least one sugar alcohol or a mixture of sugar alcohols fused onto the outside of the lozenge, and
g) at least a second portion/layer comprising nicotine salt and at least one sugar alcohol or a mixture of sugar alcohols fused onto the lozenge, (see Fig 2)
   and wherein the at least one sugar alcohol or a mixture of sugar alcohols comprised in the portion(s)/layer(s) comprises at least erythritol,
   and wherein the nicotine release from the at least one portion/layer is immediate and the nicotine release from the lozenge is extended
      and
   wherein the at least one buffer promotes a rapid uptake of nicotine through the oral mucosa.

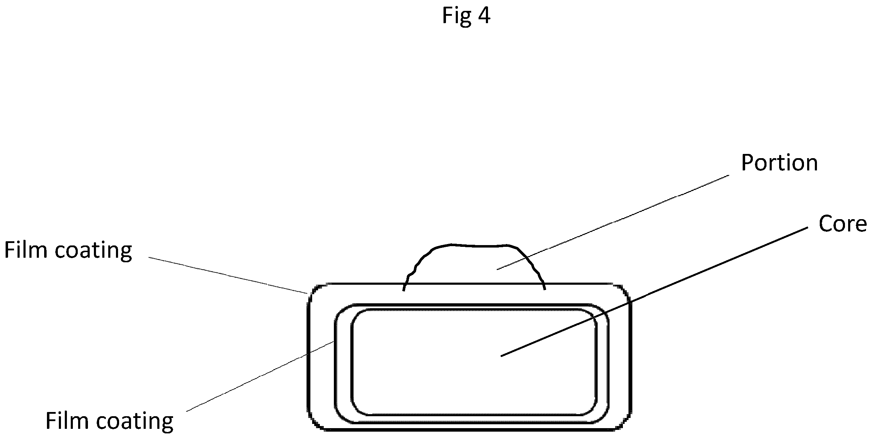

In another embodiment the lozenge comprises nicotine and at least one polymer-based film coating covering the lozenge comprising at least one flavor and at least one sweetener, and
h) at least one polymer-based film coating covering the first polymer-based film coating comprising at least one buffer and at least one sugar alcohol or a mixture of sugar alcohols fused onto the outside of the lozenge, and
i) at least a second portion/layer comprising nicotine salt and at least one sugar alcohol or a mixture of sugar alcohols fused onto the lozenge, (see Fig 4)

and wherein the at least one sugar alcohol or a mixture of sugar alcohols comprised in the portion(s)/layer(s) comprises at least erythritol,
and wherein the nicotine release from the at least one portion/layer is immediate and the nicotine release from the lozenge is extended
   and
wherein the at least one buffer promotes a rapid uptake of nicotine through the oral mucosa.

The lozenge might be direct compressed or granulated and compressed.

The lozenge core comprises nicotine, such as nicotine bound to ion exchange resins, such as nicotine polacrilex, nicotine bound to zeolites and/or nicotine bound to beta cyclodextrin, preferably in the form as nicotine polacrilex. The nicotine present in the core (calculated as the free base) may be from about 1.0 to about 6.0 mg, about 2.0 to about 4.0 mg, such as 1.0, 1.25, 1.5, 1.75, 2.0, 2.25, 2.5, 2.75, 3.0, 3.25, 3.5, 3.75, 4.0, 4.25, 4.5, 5.0, 5.5 or 6.0, calculated per piece of lozenge. The nicotine will be released from the core when the user is sucking on the lozenge and the release will occur over an extended time period, over a time up to about 10-20 minutes.

In addition, the core may comprise excipients including fillers, glidants, buffers, lubricants, sweeteners, flavors, coloring agents, binding/gelling agents and mixtures thereof well known for a person skilled in the art.

In one embodiment, at least one buffer is present in the at least one film forming polymer coating or the at least one portion/layer.

The at least one buffer is selected from the group consisting of sodium carbonate, sodium bicarbonate, potassium carbonate, potassium bicarbonate, trometamol base (Tris base), or the corresponding conjugated acid of trometamol such as Trometamol hydrochloride (Tris HCl), trisodium phosphate, disodium hydrogenphosphate, sodium dihydrogen phosphate, tripotassium phosphate, dipotassium hydrogenphosphate, potassium dihydrogen phosphate and mixtures thereof.

In some examples the at least one buffer is selected from the group consisting of sodium carbonate, sodium bicarbonate, trometamol base (Tris base) or the corresponding conjugated acid of trometamol such as Trometamol hydrochloride (Tris HCl), and mixtures thereof.

However, the acidic nicotine salt and the alkaline buffers are not suitable to be present in the same portion/layer outside the lozenge, whereas interaction during the melting, solubilization, drying or cooling process could lead to transformation of nicotine to the its' free base form which is a volatile and unstable liquid, thus resulting in loss of nicotine from the portion outside of the lozenge or a chemically unstable product.

The at least one buffer may be present in a total amount from about 1.0 to about 7.5 mg (calculated per piece of lozenge), such as about 1.0 to about 6.0 such as about 2.0 to about 6.0 or about 2.0 to about 5.0 mg, or about 3.0 to about 6.0 or about 3.0 to about 5.0, such as 1.0, 1.25, 1.5, 1.75, 2.0, 2.25, 2.5, 2.75, 3.0, 3.25, 3.5, 3.75, 4.0, 4.25, 4.5, 4.75, 5.0, 5.25, 5.5, 5.75, 6.0, 6.25, 6.5, 6.75, 7.0 7.25, or 7.5 mg.

In one example sodium carbonate or sodium bicarbonate may each be present in an amount of 0.5 mg to about 3.5 mg, such as 0.5 mg to 2.5 mg, such as 0.5 mg to 1.0 mg or 0.5, 1.0, 1.5, 2.0, or 2.5 mg and trometamol base (Tris base), or the corresponding conjugated acid of trometamol such as Trometamol hydrochloride (Tris HCl), may be present in an amount of 1.5 mg to 5.0 mg, such as 2.0 to 5.0, 3.0 mg to 5.0 mg or 1.5, 2.0, 2.5, 3.0, 3.5, 4.0, 4.5 or 5.0 mg.

The film forming polymers may be chosen among hydroxy propyl methyl cellulose (HPMC), methyl hydroxy ethyl cellulose (MHEC), hydroxy propyl cellulose (HPC), hydroxyethyl cellulose (HEC), methacrylic acid copolymer-type C, sodium carboxy methyl cellulose , Hydroxypropyl methylcellulose phthalate, (HPMCP), ethyl hydroxyl ethyl cellulose (EHEC), and other film forming polymers such as, polydextrose, polyethylene glycols, acrylate polymers, polyvinyl alcohol-polyethylene glycol graft copolymers, complex of polyvinylpyrrolidone (PVP), such as povidone, polyvinyl alcohol (PVOH or PVA), microcrystalline cellulose, carrageenan, pregelatinized starch, polyethylene glycol, and combinations thereof.

In one embodiment, the film-forming polymers are selected among hydroxy propyl methyl cellulose (HPMC), methyl hydroxy ethyl cellulose (MHEC), hydroxy propyl cellulose (HPC), hydroxyethyl cellulose (HEC), ethyl hydroxyl ethyl cellulose (EHEC) and polyvinyl alcohol (PVOH or PVA).

If two polymer-based film coatings are applied (dual polymer coating), the same as well as different polymers and mixtures thereof could be used in the two coating layers. Examples for dual polymer coating are based on hydroxy propyl methyl cellulose (HPMC) or one is based on hydroxy propyl methyl cellulose (HPMC) an the other one is based on another film forming polymer such as polyvinyl alcohol (PVOH or PVA). The different film forming polymers may have different characteristics. For example, PVA is regarded to be more resistant against elevated pH and could thus be suitable to be used for the buffer containing film coating when there is an aim for a high pH.

HPMC provides a nice palatability as well as being able to provide a prolonged boost of flavor and sweetener when applied to a lozenge.

The film coating may have an average thickness from 10 to 500 microns, more preferably from 20 to 250 microns, such as from 30 to 150 microns. The film thickness may be measured using different methods known in the art such as SEM (Scanning Electron Microscopy), digital micrometer, X-ray microtomography, terahertz pulsed imaging etc. See further e g Quantitative Analysis of Film Coating in a Pan Coater Based on In-Line Sensor Measurements, Jose D. Perez-Ramos et al, AAPS PharmSciTech 2005; 6 (1) Article 20, Nondestructive analysis of tablet coating thicknesses using terahertz pulsed imaging. J Pharm Sci. 2005; 94:177Y183. Fitzgerald A J, Cole B E, Taday P F., Hancock B, Mullarney M P. X-ray microtomography of solid dosage forms. Pharm Technol. 2005; 29:92Y100.

The lozenge, the at least one polymer-based film coating or at least one portion/layer may have additional ingredients such as at least one flavor and at least one sweetener.

In one embodiment one polymer-based coating comprises at least one buffer and another polymer-based coating at least one flavor and at least one sweetener.

Examples of flavoring agents/flavors include, fruit and berry flavors such as lime, orange, lemon, black current, blood orange, cranberry, cloudberry, goji berry, raspberry, strawberry, wild strawberry, sea buckthorn, cherry, melon, kiwi, papaya, pineapple, passion fruit, coconut, and other flavors such as honey, herbs, the, anise, water grass, lemon grass, cooling agent, ginger, coffee, eucalyptus, mangostan, peppermint, spearmint, wintergreen, tutti-frutti, cinnamon, cacao/cocoa, vanilla, liquorice, salt, pepper, chili, menthol, aniseeds, or mixtures thereof. The flavoring agents/flavors may be natural extracts as well as synthetic and semisynthetic versions as well as mixtures of flavors. The flavors may be the same or different and can be present in the lozenge, film coating(s), as well as in the outer portion(s)/layer(s). Suitable examples of flavors are mint family flavors, fruit and berry flavors.

In addition, the lozenge, has at least one artificial sweetener. The at least one artificial sweetener may be present in the lozenge, film coating(s) and/ or in the outer portion(s)/layer(s). Examples of artificial sweeteners are saccharin, sodium saccharin, aspartame, acesulfame K, neotame, thaumatin, glycyrrhizin, sucralose, cyclamate, dihydrochalcone, alitame, miraculin and monellin and mixtures thereof.

The polymer-based film coating may contain one or more plasticizer to the film-forming polymer to facilitate the spreading and film forming capability. Examples on useful plasticizers are glycerol, propylene glycol, polyethylene glycol (PEG 200-6000), organic esters e g triacetin (glyceryl triacetate), triethyl citrate, diethyl phtalate, dibutyl phtalate, dibutyl sebacete, acetyltriethyl citrate, acethyltributyl citrate, tributyl citrate, and oils/glycerides such as fractionated coconut oil, castor oil and distilled acetylated monoglycerides. Additionally, or alternatively, surfactants may be included to facilitate the incorporation of flavors and to improve penetration and spreading properties of the coating liquid. Non-limiting examples of surfactant are polysorbates derived from PEG-ylated sorbitan esterified with fatty acids such as Polysorbate 20 (Polyoxyethylene (20) sorbitan monolaurate), Polysorbate 40 (Polyoxyethylene (20) sorbitan monopalmitate), Polysorbate 60 (Polyoxyethylene (20) sorbitan monostearate), Polysorbate 80 (Polyoxyethylene (20) sorbitan monooleate) (e g Tween 80, Tween 40, Tween 20), sodium lauryl sulphate (SLS), poloxamer surfactants i.e. surfactants based on ethylene oxide--propylene oxide block copolymers and other surfactants with high HLB-value.

The portions may have the same size and weight or different sizes and weights, being placed on top of each other, beside each other or on different sides of the lozenge. The portions comprising nicotine and buffer(s) may be placed on top of each other, beside each other or on opposite sides of the lozenge. The et least one portion/layer may be deposited in a debossed smooth cavity(cavities) on the lozenge.

The above mentioned portion(s)/layer(s) may comprise a mixture of erythritol and xylitol in a proportional amount of about 90:10, 91:9, 92:8, 93:7, 96:4, 95:5, 96:4, 97:3, 98:2, 99:1 or 100:0 (% w/w of erythritol:xylitol). The portion comprises at least erythritol.

The nicotine salt is evenly distributed in the nicotine containing at least one portion/layer outside the lozenge. In those embodiments wherein there are at least one portion/layer outside the lozenge comprising buffer(s), the buffer(s) is/are evenly distributed in the portion(s)/layer(s).

Nicotine salts present in the one or more portion(s) or layer(s) on the lozenge may be present in an amount of about 0.25 to about 2.5 mg, such as 0.5 to about 1 mg or 0.25, 0.5, 0.75, 1.0, 1.5, 2.0, 2.25 or 2.5 mg. In addition, one portion has a total weight of about 2-10 % such as 2-5 % of the total weight of the lozenge.

In another embodiment, one or more portion(s)/layer(s) comprising one or more buffer(s) could include a combination of erythritol and xylitol. The at least one sugar alcohol or a mixture of sugar alcohols comprised in the portion(s)/layer(s) comprises at least erythritol. Such a portion(s)/layer(s) may have a weight up to 10% or even up to 15 % of the total weight of the lozenge.

The outer portion(s)/layer(s) may be colored. The coloring agents include lakes and dyes being approved as a food additive and examples of coloring agents are artificial colors or natural colors. One example is when the portion is defined above and may be a dot, like the dots on a lady bird.

Examples of artificial colors approved for food use in the EU include: E104: Quinoline, Yellow, E122: Carmoisine, E124: Ponceau 4R, E131: Patent Blue V and E142: Green S. In the US, the following seven artificial colorings are generally permitted in food: FD&C Blue No. 1 - Brilliant Blue FCF, E133 (blue shade), FD&C Blue No. 2 - Indigotine, E132 (indigo shade), FD&C Green No. 3 - Fast Green FCF, E143 (turquoise shade), FD&C Red No. 3 - Erythrosine, E127 (pink shade, commonly used in glacé cherries), FD&C Red No. 40 - Allura Red AC, E129 (red shade), FD&C Yellow No. 5 - Tartrazine, E102 (yellow shade), FD&C Yellow No. 6 - Sunset Yellow FCF, E110 (orange shade).

Examples of natural colors includes: Carotenoids (E160, E161, E164), chlorophyllin (E140, E141), anthocyanins (E163), and betanin (E162), Annatto (E160b), a reddish-orange dye made from the seed of the achiote, Caramel coloring (E150a-d), made from caramelized sugar, Carmine (E120), a red dye derived from the cochineal insect, *Dactylopius coccus,* Elderberry juice (E163), Lycopene (E160d), Paprika (E160c) and Turmeric (E100) or titaniumdioxide.

The nicotine polacrilex present in the core may be in an amount from about 1.0 to about 6.0 mg, about 2.0 to about 4.0 mg, such as 1.0, 1.25, 1.5, 1.75, 2.0, 2.25, 2.5, 2.75, 3.0, 3.25, 3.5, 3.75, 4.0, 4.5, 5.0, 5.5 or 6.0, calculated as the free base per lozenge and the nicotine salt present in the at least one portion/layer or at least one film coating may be in an amount of from about 0.25 to about 2.5 mg, such as 0.25, 0.3, 0.35, 0.4, 0.45, 0.5, 0.55, 0.6, 0.65, 0.7, 0.75, 0.8, 0.85, 0.9, 0.95, 1.0, 1.05, 1.1, 1.15, 1.2, 1.25, 1.3, 1.35, 1.4, 1.45, 1.5, 1.55, 1.6, 1.65, 1.7, 1.75, 1.8, 1.85, 1.9, 1.95, 2.0, 2.05, 2.1, 2.15, 2.2, 2.25, 2.3, 2.35, 2.4, 2.45 or 2.5 mg, such as 0.25 to 1.0 mg or 0.25 to 1.5 or 0.25 to about 2.0 mg (calculated as the free base).

The relative amounts of nicotine salt as well as the amounts of buffer(s) located on the surface of the lozenge are important since the buffer(s) will enable a conversion of the nicotine salt to its free base form to promote a fast uptake of nicotine.

If too little buffer(s) in relation to the nicotine salt is available, the conversion of nicotine to its free base form will be negatively affected, which may lead to a higher portion of the nicotine in its acidic form, resulting in a lower fraction of nicotine available for absorption into the oral mucosa and instead being subject to transportation into the gastrointestinal tract and exposure to the so called first-pass metabolism. This could lead to a comparably slower and reduced absorption of nicotine affecting the nicotine concentrations in the systemic circulation and a comparably slower (and lower) nicotine craving relief.

### MANUFACTURING PROCESS OF THE LOZENGE

Standard mixing equipment may be used for mixing together components of compositions of the invention. The mixing time period is likely to vary according to the equipment used, and the skilled person will have no difficulty in determining by routine experimentation a suitable mixing time for a given combination of ingredient(s). One way of producing the lozenges is found in the examples. The manufacturing process may as well comprise additional steps of granulation, drying and milling and/or sieving to obtain a lozenge.

Finally, the invention relates to the above defined lozenges for use in the treatment of a human beings suffering from cravings from tobacco dependency and/or e-cigarette dependency.

### EXAMPLES

### EXAMPLE 1

### PRODUCTION OF A NICOTINE LOZENGE

All ingredients were purchased in pharmaceutical quality except for the flavoring agents that were of food grade.

The ingredients were blended.

The blending times were optimized to obtain a homogenous powder mixture obvious for a person skilled in the art.

The lozenges were produced by direct compression of the powder mix.

The lozenge shape was oblong with targeted lozenge weight of 600 mg.

The manufacturing was performed in manufacturing area with controlled temperature and humidity.

### EXAMPLE 2

### PRODUCTION OF A COATED NICOTINE LOZENGE

The core of the lozenge was produced as in EXAMPLE 1.

The coating polymer was dispersed in warm water and then cooled down. The other raw materials were added into the coating solution.

The coating solution was homogenized.

The cores were then filmcoated and the film coating was controlled on the outlet air temperature of 45°C.

### EXAMPLE 3

Examples for single polymer coating of lozenge

| **Example** | **1** | **2** | **3** | **4** | **5** | **6** | **7** |
|---|---|---|---|---|---|---|---|
| | *Amounts expressed in %*/*% w*/*w per excipient per batch* | | | | | | |
| Hydroxypropyl methylcellulose (HPMC) | 15 | 16 | 25 | 26 | 26 | 26 | 26 |
| Polysorbate 80 | 0,2 | 0,1 | 0,3 | 0,1 | 0,2 | 0,1 | 0,3 |
| Sucralose | 2 | 3 | 2 | 3 | 2 | 3 | 3 |
| Flavor | 3 | 2 | 3 | 4 | 3 | 3 | 3 |
| Sodium carbonate | 0,5 | | 2,5 | 3 | 2,5 | 2,5 | 4 |
| Sodium bicarbonate | | 1 | 2,5 | 1 | 2,5 | | 1 |
| Sodium hydrogen phosphate | | | | | 5 | 5 | 4 |
| Trometamol | 5 | 5 | 7 | 6 | | | |
| Purified water¹⁾ | q.s. | q.s. | q.s. | q.s. | q.s. | q.s. | q.s. |
| Hydrochloric acid (10%)²⁾ | ad pH 9 | ad pH 9 | ad pH 10 | ad pH 10,4 | ad pH 9,9 | ad pH 10,7 | ad pH 10,4 |

| | | | | | | | |
|---|---|---|---|---|---|---|---|
| ¹⁾ Majority of purified water is removed during film coating process ²⁾ Coating preparation is pH adjusted with Hydrochloric (10%) acid to target pH | | | | | | | |

### EXAMPLE 4

Examples for dual polymer coating of lozenge

| | **Example** | **1** | **2** | **3** | **4** | **5** | **6** | **7** | **8** | **9** |
|---|---|---|---|---|---|---|---|---|---|---|
| **Polymer coating A** | Polyvinylalcohol (PVA) | 8 | 12 | 15 | 17 | 12 | 15 | 10 | 17 | 16 |
| | Sodium carbonate | 1 | 1 | 0,5 | | 2,5 | 3 | 2,5 | 2,5 | 4 |
| | Sodium bicarbonate | | | | 1 | 2,5 | 1 | 2,5 | | 1 |
| | Sodium hydrogen phosphate | | | | | | | 5 | 5 | 4 |
| | Trometamol | 3 | 5 | 5 | 5 | 7 | 6 | | | |
| | Purified water¹⁾ | q.s. | q.s. | q.s. | q.s. | q.s. | q.s. | q.s. | q.s. | q.s. |
| | Hydrochloric acid (10%)²⁾ | ad pH 9 | ad pH 9 | ad pH 9 | ad pH 9 | ad pH 10 | ad pH 10,4 | ad pH 9,9 | ad pH 10,7 | ad pH 10,4 |
| **Polymer coating B** | Hydroxypropylmethylcellulose (HPMC) | 13 | 14 | 15 | 16 | 15 | 16 | 17 | 18 | 17 |
| | Polysorbate 80 | 0,1 | 0,2 | 0,2 | 0,1 | 0,3 | 0,1 | 0,2 | 0,1 | 0,3 |
| | Sucralose | 2 | 3 | 2 | 3 | 2 | 3 | 2 | 3 | 3 |
| | Flavor | 2,5 | 3 | 3 | 2 | 3 | 4 | 3 | 3 | 3 |
| | Purified water¹⁾ | q.s. | q.s. | q.s. | q.s. | q.s. | q.s. | q.s. | q.s. | q.s. |

| | | | | | | | | | | |
|---|---|---|---|---|---|---|---|---|---|---|
| 1) Majority of purified water is removed during film coating process 2) Coating preparation is pH adjusted with Hydrochloric (10%) acid or NaOH (0.1 M) to target pH | | | | | | | | | | |

Dry content of coating preparation can be varied between 20 - 50 w/w% to facilitate the incorporation of various of amounts of buffering agents.

### EXAMPLE 5

Preparation of different mixtures of portions containing nicotine and excipients (examples 1, 2 and 5 are provided for reference)

| | **Examples** | | | | | | | |
|---|---|---|---|---|---|---|---|---|
| | **1** | **2** | **3** | **4** | **5** | **6** | **7** | **8** |
| | *Amounts expressed in % w*/*w per component per batch* | | | | | | | |
| **APIs** | | | | | | | | |
| Nicotine Bitartrate Dihydrate | 15,39 | 15,4 | 14,3 | 14,3 | 14,3 | 14,3 | 14,3 | 7,13 |
| **Excipients** | | | | | | | | |
| Xylitol | | 82,8 | 80,4 | | 82,4 | 20,0 | 3,9 | |
| Isomalt | 84,61 | | | | | | | |
| Erythritol | | | 3,3 | 83,7 | | 65,7 | 79,8 | 92,7 |
| Dye | q.s. | q.s. | q.s. | q.s. | q.s. | q.s. | q.s. | q.s. |
| Mannitol 25 µ | | | | | 3,3 | | | |
| Sodium Carbonate | | | | | | | | |
| Neotame | | | | | | | | 0,15 |
| Titanium dioxide | | 1,8 | 2,0 | 2,0 | | | 2 | |

Manufacturing: all starting materials, polyols or mixtures of several polyols, nicotine source and dye, seeding agent or sweetener, if applicable where mixed in a glass beaker. The mixture was heated until melting under stirring of all the components except for titanium dioxide. 20 to 40 mg of the melted mass was deposited on the lozenge with aid of a micropipette. The droplet was occasionally flattened with a heated tool directly after deposition to reduce the thickness of the deposited droplet.

Use of Xylitol as the primary polyol gave too long solidification times to be utilized in commercial manufacturing. Use of seeding agents (Mannitol 25 µ or Titanium dioxide) did not shorten the solidification time. Isomalt had very fast solidification time but very high melting point (153° C) which is not desirable from stability and safety perspective. Erythritol exhibited fast solidification and much lower melting temperature (121,5° C).

### EXAMPLE 6

### Buffer-containing portions.

| **Example** | **1** | **2** | **3** | **4** | **5** | **6** | **7** | **8** |
|---|---|---|---|---|---|---|---|---|
| | *Amounts expressed in (g) per excipient per batch* | | | | | | | |
| Erythritol | 29,50 | 29,250 | 28,875 | 28,50 | 29,250 | 28,50 | 28,875 | 28,50 |
| Sodium Carbonate anhydrous | 0,50 | 0,750 | 1,125 | 1,50 | | | | |
| Sodium Bicarbonate | | | | | 0,750 | 1,50 | | |
| Potassium Carbonate | | | | | | | 1,125 | |
| Trometamol | | | | | | | | 1,50 |

Procedure: all starting materials, polyol and buffer(s) where mixed in a glass beaker. The mixture was heated until melting under stirring of all the components except for titanium dioxide. 20 to 40 mg of the melted mass was deposited on the coated lozenge with aid of a micropipette. The droplet was occasionally flattened with a heated tool directly after deposition to reduce the thickness of the deposited droplet.

### EXAMPLE 7

| | 1 | 2 | 3 | 4 | 5 |
|---|---|---|---|---|---|
| | *Amounts expressed in (g) per excipient per batch* | | | | |
| Erythritol (g) | 29,25 | 28,875 | 28,5 | 27,00 | 26,75 |
| Trometamol (g) | 0,75 | 1,125 | 1,5 | 3,00 | 3,75 |
| Na₂CO₃ (g) | 0,75 | 0,75 | 0,75 | 0,75 | 0,75 |
| Total (g) | 30,75 | 30,75 | 30,75 | 30,75 | 30,75 |

All example formulations 1 to 5 gained a clear solution upon gentle manual mixing and melting at 125°C without any remaining residue particles by visual control. Solidification time upon deposition of portions on the lozenge was prolonged with increasing concentration of Trometamol but within limits for manufacturability.

Manufacturing: all starting materials, polyol and buffer(s) where mixed in a glass beaker. The mixture was heated until melting under stirring of all the components except for titanium dioxide. 20 to 40 mg of the melted mass was deposited on the lozenge with aid of a micropipette. The droplet was occasionally flattened with a heated tool directly after deposition to reduce the thickness of the deposited droplet.

## Claims

1. A lozenge comprising nicotine within the core
and
i) at least a first portion/layer comprising at least one buffer and at least one sugar alcohol or a mixture of sugar alcohols fused onto the outside of the lozenge, and
ii) at least a second portion/layer comprising nicotine salt and at least one sugar alcohol or a mixture of sugar alcohols fused onto the lozenge,
or
iii) at least one polymer-based film coating covering the lozenge comprising at least one buffer and at least one flavor and at least one sweetener, and
iv) at least one portion/layer comprising nicotine salt and at least one sugar alcohol or a mixture of sugar alcohols fused onto the lozenge,
or
v) at least one polymer-based film coating covering the lozenge comprising at least one flavor and at least one sweetener, and
vi) at least a first portion/layer comprising at least one buffer and at least one sugar alcohol or a mixture of sugar alcohols fused onto the outside of the lozenge, and
vii) at least a second portion/layer comprising nicotine salt and at least one sugar alcohol or a mixture of sugar alcohols fused onto the lozenge,
and wherein the at least one sugar alcohol or a mixture of sugar alcohols comprised in the portion(s)/layer(s) comprises at least erythritol,
and wherein the nicotine release from the at least one portion/layer is immediate and the nicotine release from the core is extended,
and wherein the at least one buffer promotes a rapid uptake of nicotine through the oral mucosa.

2. The lozenge according to claim 1, wherein the at least one buffer is selected from the group consisting of sodium carbonate, sodium bicarbonate, potassium carbonate, potassium bicarbonate, Trometamol-base (Tris-base)Trometamol-hydrochloride (Tris-HCl), Tris (hydroxymethyl) amino methane, trisodium phosphate, disodium hydrogenphosphate, sodium dihydrogen phosphate, tripotassium phosphate, dipotassium hydrogenphosphate, potassium dihydrogen phosphate, sodium citrate and potassium citrate and mixtures thereof.

3. The lozenge according to claim 2, wherein the at least one buffer is selected from the group consisting of sodium carbonate, sodium bicarbonate, trometamol base (Tris base) Trometamol- hydrochloride (Tris-conjugated acid) or mixtures thereof.

4. The lozenge according to any of preceding claims wherein at least one buffer is present in an amount from about 1.0 to about 7.5 mg/lozenge (calculated as the free base).

5. The lozenge according to claims 1-4, wherein the nicotine in the core is nicotine polacrilex and nicotine in the portion/layer is nicotine bitartrate or nicotine ditartrate dihydrate.

6. The lozenge according to any of preceding claims, wherein the core comprises nicotine in an amount of from about 1.0 mg to about 7.5 mg and at least one portion/layer comprises nicotine salt present in an amount of from about 0.25 mg to about 2.5 mg (calculated as the free base).

7. The lozenge according to any of preceding claims, wherein the polymer-based film is selected from the group consisting of hydroxy propyl methyl cellulose (HPMC), methyl hydroxy ethyl cellulose (MHEC), hydroxy propyl cellulose (HPC), hydroxyethyl cellulose (HEC), ethyl hydroxyl ethyl cellulose (EHEC) and polyvinyl alcohol (PVOH or PVA).

8. The lozenge according to any of preceding claims, wherein the lozenge is direct compressed or granulated and compressed.

9. The lozenge according to any of preceding claims, wherein the at least one sugar alcohol or a mixture of sugar alcohols comprised in the portion(s)/layer(s) comprise a mixture of erythritol and xylitol in a proportional amount of about 90:10, 91:9, 92:8, 93:7, 96:4, 95:5, 96:4, 97:3, 98:2, 99:1 or 100:0 % w/w of erythritol:xylitol.

10. The lozenge according to any of claims 1-9 for use in the treatment of a human being suffering from cravings from tobacco and/or e-cigarette dependency.

## Patentansprüche

1. Lutschtablette, umfassend Nicotin im Kern und
i) mindestens eine erste mindestens einen Puffer und mindestens einen Zuckeralkohol oder eine Mischung von Zuckeralkoholen umfassende Portion/Schicht, die an der Außenseite der Lutschtablette ankondensiert ist, und
ii) mindestens eine zweite Nicotinsalz und mindestens einen Zuckeralkohol oder eine Mischung von Zuckeralkoholen umfassende Portion/Schicht, die an die Lutschtablette ankondensiert ist,
oder
iii) mindestens eine die Lutschtablette bedeckende polymerbasierte Filmbeschichtung, die mindestens einen Puffer und mindestens einen Aromastoff und mindestens einen Süßstoff umfasst, und
iv) mindestens eine Nicotinsalz und mindestens einen Zuckeralkohol oder eine Mischung von Zuckeralkoholen umfassende Portion/Schicht, die an die Lutschtablette ankondensiert ist,
oder
v) mindestens eine die Lutschtablette bedeckende polymerbasierte Filmbeschichtung, die mindestens einen Aromastoff und mindestens einen Süßstoff umfasst, und
vi) mindestens eine erste mindestens einen Puffer und mindestens einen Zuckeralkohol oder eine Mischung von Zuckeralkoholen umfassende Portion/Schicht, die an der Außenseite der Lutschtablette ankondensiert ist, und
vii) mindestens eine zweite Nicotinsalz und mindestens einen Zuckeralkohol oder eine Mischung von Zuckeralkoholen umfassende Portion/Schicht, die an die Lutschtablette ankondensiert ist,
und wobei der mindestens eine in der Portion/den Portionen/der Schicht/den Schichten enthaltene Zuckeralkohol oder eine Mischung von Zuckeralkoholen mindestens Erythrit umfasst,
und wobei die Nicotinfreisetzung aus der mindestens einen Portion/Schicht unmittelbar erfolgt und die Nicotinfreisetzung aus dem Kern retardiert erfolgt und wobei der mindestens eine Puffer eine schnelle Aufnahme von Nicotin über die Mundschleimhaut fördert.

2. Lutschtablette nach Anspruch 1, wobei der mindestens eine Puffer aus der aus Natriumcarbonat, Natriumhydrogencarbonat, Kaliumcarbonat, Kaliumhydrogencarbonat, Trometamol-Base (Tris-Base), Trometamol-Hydrochlorid (Tris-HCl), Tris(hydroxymethyl)aminomethan, Natriumphosphat, Dinatriumhydrogenphosphat, Natriumdihydrogenphosphat, Kaliumphosphat, Dikaliumhydrogenphosphat, Kaliumdihydrogenphosphat, Natriumcitrat und Kaliumcitrat sowie Mischungen davon bestehenden Gruppe ausgewählt ist.

3. Lutschtablette nach Anspruch 2, wobei der mindestens eine Puffer aus der aus Natriumcarbonat, Natriumhydrogencarbonat, Trometamol-Base (Tris-Base), Trometamol-Hydrochlorid (Tris-HCl) oder Mischungen davon bestehenden Gruppe ausgewählt ist.

4. Lutschtablette nach einem der vorhergehenden Ansprüche, wobei mindestens ein Puffer in einer Menge von etwa 1,0 bis etwa 7,5 mg/Lutschtablette (berechnet als freie Base) vorhanden ist.

5. Lutschtablette nach einem der Ansprüche 1-4, wobei das Nicotin in dem Kern Nicotin-Polacrilex ist und das Nicotin in der Portion/Schicht Nicotinhydrogentartrat oder Nicotinditartrat-dihydrat ist.

6. Lutschtablette nach einem der vorhergehenden Ansprüche, wobei der Kern Nicotin in einer Menge von etwa 1,0 mg bis etwa 7,5 mg umfasst und mindestens eine Portion/Schicht Nicotinsalz umfasst, das in einer Menge von etwa 0,25 mg bis etwa 2,5 mg (berechnet als freie Base) vorhanden ist.

7. Lutschtablette nach einem der vorhergehenden Ansprüche, wobei der polymerbasierte Film aus der aus Hydroxypropylmethylcellulose (HPMC), Methylhydroxyethylcellulose (MHEC), Hydroxypropylcellulose (HPC), Hydroxyethylcellulose (HEC), Ethylhydroxylethylcellulose (EHEC) und Polyvinylalkohol (PVOH oder PVA) bestehenden Gruppe ausgewählt sind.

8. Lutschtablette nach einem der vorhergehenden Ansprüche, wobei die Lutschtablette direkt komprimiert oder granuliert und komprimiert ist.

9. Lutschtablette nach einem der vorhergehenden Ansprüche, wobei der mindestens eine in der Portion/den Portionen/der Schicht/den Schichten enthaltene Zuckeralkohol oder eine Mischung von Zuckeralkoholen eine Mischung von Erythrit und Xylit in einer proportionalen Menge von etwa 90:10, 91:9, 92:8, 93:7, 96:4, 95:5, 96:4, 97:3, 98:2, 99:1 oder 100:0 Gew.-% Erythrit:Xylit umfasst.

10. Lutschtablette nach einem der Ansprüche 1-9 zur Verwendung bei der Behandlung eines Menschen, der an einem Verlangen nach Tabak und/oder E-Zigaretten-Abhängigkeit leidet.

## Revendications

1. Pastille comprenant de la nicotine dans le noyau et
i) au moins une première portion/couche comprenant au moins un tampon et au moins un alcool de sucre ou un mélange d'alcools de sucre fondu sur l'extérieur de la pastille, et
ii) au moins une deuxième portion/couche comprenant un sel de nicotine et au moins un alcool de sucre ou un mélange d'alcools de sucre fondu sur la pastille,
ou
iii) au moins un pelliculage à base de polymère recouvrant la pastille comprenant au moins un tampon et au moins un arôme et au moins un édulcorant, et
iv) au moins une portion/couche comprenant un sel de nicotine et au moins un alcool de sucre ou un mélange d'alcools de sucre fondu sur la pastille,
ou
v) au moins un pelliculage à base de polymère recouvrant la pastille comprenant au moins un arôme et au moins un édulcorant, et
vi) au moins une première portion/couche comprenant au moins un tampon et au moins un alcool de sucre ou un mélange d'alcools de sucre fondu sur l'extérieur de la pastille, et
vii) au moins une deuxième portion/couche comprenant un sel de nicotine et au moins un alcool de sucre ou un mélange d'alcools de sucre fondu sur la pastille,
et dans laquelle l'alcool de sucre ou le mélange d'alcools de sucre compris dans la (ou les) portion(s)/couche(s) comprend au moins l'érythritol,
et dans laquelle la libération de nicotine depuis la portion/couche est immédiate et la libération de nicotine du noyau est prolongée,
et dans laquelle le tampon favorise une absorption rapide de la nicotine à travers la muqueuse orale.

2. Pastille selon la revendication 1, dans laquelle le tampon est choisi dans un groupe constitué du carbonate de sodium, du bicarbonate de sodium, du carbonate de potassium, du bicarbonate de potassium, du trométamol-base (tris-base), du chlorhydrate de trométamol (tris-HCl), du tris(hydroxyméthyl)aminométhane, du phosphate trisodique, de l'hydrogénophosphate disodique, du dihydrogénophosphate de sodium, du phosphate tripotassique, de l'hydrogénophosphate dipotassique, du dihydrogénophosphate de potassium, du citrate de sodium et du citrate de potassium et de leurs mélanges.

3. Pastille selon la revendication 2, dans laquelle le tampon est choisi dans un groupe constitué du carbonate de sodium, du bicarbonate de sodium, du trométamol-base (tris-base), du chlorhydrate de trométamol (tris-acide conjugué) ou des mélanges correspondants.

4. Pastille selon l'une quelconque des revendications précédentes, dans laquelle au moins un tampon est présent en une quantité d'environ 1,0 à environ 7,5 mg/pastille (calculée comme base libre).

5. Pastille selon les revendications 1 à 4, dans laquelle la nicotine dans le noyau est de la nicotine polacrilex et la nicotine dans la partie/couche est du bitartrate de nicotine ou du ditartrate de nicotine dihydraté.

6. Pastille selon l'une quelconque des revendications précédentes, dans laquelle le noyau comprend de la nicotine en une quantité allant d'environ 1,0 mg à environ 7,5 mg et au moins une portion/couche comprend un sel de nicotine présent en une quantité d'environ 0,25 mg à environ 2,5 mg (calculée comme base libre).

7. Pastille selon l'une quelconque des revendications précédentes, dans laquelle le film à base de polymère est choisi dans un groupe constitué de l'hydroxypropyl-méthyl-cellulose (HPMC), du méthylhydroxyéthyl-cellulose (MHEC), de l'hydroxypropyl-cellulose (HPC), de l'hydroxyéthyl-cellulose (HEC), de l'hydroxyéthyl-éthyl-cellulose (EHEC) et du poly(alcool vinylique) (PVOH ou PVA) ou d'un mélange correspondant.

8. Pastille selon l'une quelconque des revendications précédentes, dans laquelle la pastille est comprimée directement ou granulée et comprimée.

9. Pastille selon l'une quelconque des revendications précédentes, dans laquelle l'alcool de sucre ou le mélange d'alcools de sucre compris dans la (ou les) portion(s)/couche(s) comprend un mélange d'érythritol et de xylitol en une quantité proportionnelle d'environ 90:10, 91:9, 92:8, 93:7, 96:4, 95:5, 96:4, 97:3, 98:2, 99:1 ou 100:0 % p/p d'érythritol:xylitol.

10. Pastille selon l'une quelconque des revendications 1 à 9 pour une utilisation dans le traitement d'un humain souffrant d'envies liées à une dépendance au tabac et/ou à la cigarette électronique.
